# EUROPEAN PATENT SPECIFICATION

(11) **EP 3 154 523 B1**
(45) Date of publication and mention of the grant of the patent: **21.11.2018**
(21) Application number: 15741328.7
(22) Date of filing: 15.06.2015
(51) Int. Cl.: A61K 9/16, A61K 9/50, A61K 31/606

(54) **FORMULATION FOR ORAL ADMINISTRATION CONTAINING MESALAZINE**
FORMULIERUNG ZUR ORALEN VERABREICHUNG MIT MESALAZIN
FORMULATION POUR ADMINISTRATION PAR VOIE ORALE CONTENANT DE LA MÉSALAZINE

(30) Priority: 16.06.2014 IT FI20140148
(43) Date of publication of application: 19.04.2017
(73) Proprietor: VALPHARMA INTERNATIONAL S.P.A., 47864 Pennabilli (IT)
(72) Inventor: VALDUCCI, Roberto, 47039 Savignano Sul Rubicone (IT); AVANESSIAN, Serozh, 47827 Villa Verucchio (IT)
(74) Representative: Valenza, Silvia
(86) International application number: PCT/IB2015/054506
(87) International publication number: WO 2015/193788

(56) References cited:
- WO-A1-92/16206
- WO-A1-03/032952
- WO-A1-2010/077908
- US-A1- 2003 152 627
- US-A1- 2007 043 004
- DATABASE WPI Section Ch, Week 201244 Thomson Scientific, London, GB; Class A96, AN 2012-B67034 XP002735921, LIU Q; MU X; SHI Z; ZHAO J: "Slow control released medical granules preparation for treating intestine disease, comprises pill core including 5-aminosalicylic acid, and separating layer making surface of small granule smooth and round", -& CN 102 319 218 A (BEIWOTE PHARM TECHN SHANGHAI CO LTD; PIVOT PHARM SHANGHAI CO LTD) 18 January 2012 (2012-01-18)
- WATTS P J ET AL: "Encapsulation of 5-aminosalicylic acid into Eudragit RS microspheres and modulation of their release characteristics by use of surfactants", JOURNAL OF CONTROLLED RELEASE, ELSEVIER, AMSTERDAM, NL, vol. 16, no. 3, 1 August 1991 (1991-08-01) , pages 311-318, XP025567320, ISSN: 0168-3659, DOI: 10.1016/0168-3659(91)90008-2 [retrieved on 1991-08-01]
- SWAPNA A ET AL: "Formulation and Evaluation of Mesalamine Microspheres for Colon Targeted Drug Delivery System", JOURNAL OF PHARMACY RESEARCH, vol. 4, no. 6, June 2011 (2011-06), pages 1670-1672, XP002735922, ISSN: 0974-6943
- BAREA M J ET AL: "Encapsulation of Liposomes within pH Responsive Microspheres for Oral Colonic Drug Delivery", INTERNATIONAL JOURNAL OF BIOMATERIALS, vol. 2012, 2012, pages 1-9, XP002735923,

## Description

### FIELD OF THE INVENTION

The present invention relates to the pharmaceutical compositions field, in particular it relates to formulations for the oral administration of Mesalazine in multi-particulate, multi-layer form.

### BACKGROUND OF THE INVENTION

Mesalazine, also known as 5-Aminosalicylic Acid, generally abbreviated as 5-ASA, is an Active Substance that is now widely known in that it now is the best medicinal product in the treatment of inflammatory states of the colon, and is the best adjuvant in maintenance and improvement therapies of ulcerative colitis and Crohn's disease. As Acetylsalicylic Acid derivative, it maintains its anti-inflammatory characteristics but has the advantage of acting only at topical intestinal level, avoiding systemic absorption; hence the need to formulate preparations that have a specific release only at the area of action, i.e. the colon.

Present on the market in different pharmaceutical forms, it is characterised by having high doses (but low concentration of the Active Substance, generally equal to 500-550 mg of Mesalazine per g of formulation), and formulations containing 1-2 g of the Active Substance are frequent. This very often worsens patient compliance in taking the medicinal product, as the total quantity of medicinal product is either too high, or divided into several doses. The object of the present invention is thus to provide a formulation having a high concentration of Mesalazine, to decrease the total quantity to be taken and to improve patient acceptability.

EP1178781 B1 describes a formulation containing Mesalazine that is exclusively released in the colon. It is a multi-layer formulation comprising:
a core containing the Active Substance, an inner membrane containing a pH-independent retardant polymer and an outer membrane containing a pH-dependent polymer that exclusively releases in the colon.
- Core: contains about 50% API; and consists of neutral granules (0.5-0.6 mm) that are coated, by spraying in a Fluid Bed apparatus, with a layer containing API (Mesalazine, Lactose and Aerosil) and a binder (Kollidon 25 and water).
- Inner Membrane: membrane comprising a polymer or a mixture of methacrylic acid-derived polymers (Eudragit RS, Eudragit RL), with the characteristic of having a dissolution that is slow and independent of the pH.
- Outer Membrane: membrane comprising a polymer or a mixture of methacrylic acid-derived polymers (Eudragit FS 30 D), with the characteristic of having a dissolution that is pH-dependent, exclusively in the colon.

CN 102 319 218 A discloses pellets comprising a core prepared by extruding and spheronizing polyvinyl pyrrolidone, microcrystalline cellulose, magnesium stearate and Mesalazine in the presence of water. The resulting core is first coated with a sealing layer comprising polyvinyl pyrrolidone or HPMC, then with a slow release layer comprising ethyl cellulose, and, finally, with an enteric layer comprising an acrylic resin. The cores described in examples 2 and 4 of CN 102 319 218 A have ratios of Mesalazine/excipients equal to 3/1 and 1.22/1, respectively.

Another problem often found in formulations containing Mesalazine is ensuring stability of the Active Substance over time; the development of impurities during stability studies is indeed frequent. The impurities are generally due to the oxidation that occurs when the Active Substance remains in contact with water. In fact, the membranes used to confer topical release are suspended in water, a small part of which remains present in the formulation even after drying. The cause of the degradation of Mesalazine over time is this very small part of water. The object of the present invention is to provide a formulation that can overcome this problem, guaranteeing the stability of Mesalazine over time white ensuring topical intestinal absorption in the colon.

### SUMMARY OF THE INVENTION

The present invention resolves the above-mentioned problems by means of granules containing Mesalazine as Active Pharmaceutical Ingredient (API), said granules consisting of API in mixture with a dried gelled composition in a ratio of between 97:3 and 99:1, referring to the dry portion of the composition; said granules being obtained by extrusion, spheronization and drying of a mixture of API with a gelled composition consisting of a mixture of 5-10% Polyvinylpyrrolidone, 20-40% Polysorbate and 45-75% Water, where the% relate to the percentages by weight with respect to the total weight of the gelled composition.

The granules in question are produced according to the extrusion+spheronization technique; the procedures known to the state of the art include the use of a percentage of plastic matrix, such as microcrystalline Cellulose, ranging between 30% and 50%; this gives the compound a suitable consistency for being extruded and spheronized. From here it can be deduced that the assay of the granules will be low and may not exceed 70%.

It has however been surprisingly found that by mixing just the API with the gel, in a ratio ranging between 97:3 and 99:1, preferably between 97.5:2.5 and 98.5:1.5, even more preferably 98:2 (relating to the dry weight of the substance), to then extrude and spheronize the compound obtained, compact granules with a high assay are obtained without the need to add plastic material.

Another surprising finding is that the gelled composition gives the compound a plasticity and malleability such as to be extruded with a very small mesh, so as to be able to obtain granules with a very low granulometry that are generally not obtainable according to traditional state of the art techniques. The density of the granules, which reaches 0.89 g/ml, is thus also increased with the consequent possibility of inserting in a capsule a large amount of Mesalazine per unit volume. Moreover, the gelled composition in the granules according to the invention increases the binding effect and improves the stability of the API, acting as antioxidant over time; in fact, contrary to other formulations that require the addition of anti-oxidants, a formulation comprising the granules according to the invention maintains stability over time without the addition of further ingredients. It is therefore understood that the use of polysorbate in the pellet formulation is not only merely one of the many possible excipients known in the state of the art, but has a fundamental importance, as it serves to create the gelling solution, which not only allows pellets having a very high assay to be produced, but is also responsible for the stability thereof over time.

It is also surprising that the water used for the gelled composition does not disturb the stability of the API. After extrusion and spheronization, the granules are dried immediately and in a prolonged manner to eliminate all of the water, nonetheless a very small fraction nevertheless remains within them. However, it has been surprisingly noted that the granules maintain an unexpected stability over time, produced by the antioxidant effect of the gelling substance used for granulation. Thus in one aspect, the present invention relates to a pharmaceutical formulation comprising the above-mentioned granules, it in particular relates to multi-layer pellets comprising the afore-mentioned granules as inner core.

Multi-layer pellets according to the invention comprise:
an inner core consisting of the above-mentioned granules;
a first inner coating membrane that surrounds and comes into contact with the core, said first membrane being pH-independent comprising a cellulose-derived polymer, dissolved in a non-aqueous solvent;
a second outer coating membrane that surrounds the first membrane, said second membrane being gastroprotective pH-dependent comprising a polymer derivative of methacrylic acid selected from the anionic polymers with methacrylic acid as the functional group.

To overcome the problem of the development of impurities caused by the residual water deriving from the gastro-resistant coats, the multi-layer pellets of the present invention are characterised by a first protective coat of the cores with a membrane in a non-aqueous solution. The fact that the first coating is applied in a non-aqueous environment, combined with the surprising stability of the cores produced by the gelling substance, leads to an even higher final stability, since there is no contact with water during coating.

The above-mentioned pellets are externally coated with a polymer derivative of methacrylic acid (generally Eudragit FS 30 D) to guarantee a release of the API exclusively in the colon; in fact, the chemical structure of the polymer ensures that it only dissolves in environments with a pH greater than 7.2, and this is only found in the last part of the intestine. The multi-layer pellets will pass through both the stomach and the first part of the intestine intact, to then release the medicinal product only in the last section.

An unexpected synergistic interaction between the two membranes has been surprisingly noted. Indeed, in addition to the protective effect of the first membrane and the gastro-resistant effect of the second membrane, it has been found that the two act together to regulate the release profile.

Ethyl cellulose being a pH-independent retardant polymer, it also acts here as a retardant in the release of the Active Substance, trapping for a certain period of time most of the Mesalazine within the pellets, for a period which fully or partially coincides with the time it takes the intestine to carry the medicinal product to the colon. The topical release effect in the colon, produced by the Eudragit of the second membrane, is also added to this retarding effect. An exclusive release of Mesalazine in the site of action is thus obtained. In addition thereto, the greatest advantage is that the medicinal product having already been retarded by the first membrane, the amount of second membrane to be applied will be extremely reduced, thus leading to a greater final assay of the pellets being obtained. This aspect, together with the preceding concept of starting cores having an extremely high assay, leads to a finished product with a surprisingly high amount of Mesalazine, higher than 90%, being obtained.

Thus in one aspect, the present invention relates to a pharmaceutical formulation for oral administration of Mesalazine, said formulation comprising the above-mentioned multi-layer pellets; in particular a pharmaceutical formulation comprising the above-mentioned multi-layer pellets, wherein said formulation is in the form of sachet, tablet or capsule.

### DETAILED DESCRIPTION OF THE INVENTION

For one aspect, the present invention relates to a process for preparing the granules according to the invention, said process comprising preparing the gelled composition by first dissolving Polyvinylpyrrolidone in water and then, on dissolution, adding Polysorbate to achieve the gelification. The gelled composition is then added to and mixed with Mesalazine, and finally the compound is extruded, spheronized and dried.

The gelled composition before having added it to Mesalazine is indeed a dense composition with a viscosity ranging between 480 mPa.s (4800 mPs) and 520 mPa.s (5200 mPs). This gelled composition is added to and mixed with Mesalazine, preferably in a Z-arm kneader.

After extruding and spheronizing, drying is preferably carried out in a Fluid Bed by means of the inlet of hot air at 80°C until the product reaches 50°C. The result is a starting core containing Mesalazine in a percentage greater than 97%.

Extrusion preferably takes place with a net having mesh 400-600 µm mesh.

The granules obtained according to the invention have an average size ranging between 0.4 mm and 2 mm in diameter, preferably 0.45 mm - 1.5 mm, more preferably 0.5 mm - 0.8 mm.

The first protective membrane comprises a polymer, preferably Ethyl cellulose having a viscosity of between 3 and 110 mPa.s (cPs), and a solvent, preferably Acetone, Ethanol and mixtures thereof; plasticisers, such as Triethyl citrate, Dibutyl sebacate or Polyethylene glycol and anti-sticking agents, such as Talc, Magnesium stearate, colloidal anhydrous silica or sodium stearyl fumarate, can be optionally present.

The viscosity of the Ethyl cellulose was calculated on 5% solutions in Toluene/Ethanol (80%:20%) measured at 25°C in a Ubbelohde viscometer.

The polymer is present in solution in a percentage ranging between 1% and 10%, preferably between 3% and 8%, more preferably still between 5% and 6%.

In the present invention, the multi-layer pellets are prepared by a process comprising: coating the inner core with the first membrane in a Fluid Bed or in a Coating Pan until an increase in weight ranging between 0.2% and 2% of the dry weight is achieved with respect to the weight of the cores, preferably 0.5% - 1.5%, even more preferably 0.8% - 1.2%; and coating with the second membrane in a Fluid Bed or in a Coating Pan until achieving an increase in weight from 5% to 15% of dry portion with respect to the weight of the cores, preferably 6% - 12%, even more preferably 7% - 9%.

The second outer, gastroprotective membrane comprises a polymer derivative of methacrylic acid and water; plasticisers, such as Triethyl citrate and Polysorbate, and anti-sticking agents, such as talc and glyceryl monostearate, can be optionally present. The polymer derivative of methacrylic acid is selected from anionic polymers with methacrylic acid as functional group such as, for example, Eudragit L100-55, Eudragit L 30 D-55, Eudragit L100, Eudragit L12.5, Eudragit S100, Eudragit S12.5, Eudragit FS 30 or mixtures thereof; they can sometimes also be mixed with Eudragit NE30D and Eudragit NE40D to adjust the gastro-resistance thereof.

The polymer derivative of methacrylic acid is present in suspension in a percentage ranging between 12% and 28%, preferably between 15% and 25 %, preferably between 16% and 20 %.

The resulting pellets can be sold in sachets or, given the high percentage of Active Substance, even encapsulated or compressed.

The present invention therefore relates to a pharmaceutical formulation comprising the above-mentioned multi-layer pellets, said formulation in the form of tablets or capsules.

The pellets of the invention can safely be mixed with Cellulose, and compressed with 400 mg - 500 mg - 800 mg dosages.

Particularly advantageous, however, is the possibility of encapsulating in a single dose an amount of Mesalazine equal to 500 mg, which is the generally most common daily dose. Unlike the other formulations present in literature, these pellets, having a medium to high assay (> 90% or 900 mg/g) and a high density (> 0.89 g/ml), have a very low specific weight per unit of Active Substance and can therefore be inserted into a 500 mg size 0 Capsule without any problems. This avoids the problem of multi-dose administrations, which would worsen patient compliance.

A preferred embodiment of the invention provides for the encapsulation of 500 mg of pellets in a single capsule.

The present invention will be better understood in the light of the following embodiments.

### EXPERIMENTS

### REFERENCE

EXAMPLE A-Classic reference formulation of the state of the art The composition of SALOFALK RTM GRANU-STIX, also indicated in the patent under the name of CLASSIC FORMULATION, is specified hereunder.

### SALOFALK RTM GRANU-STIX 3g

| **COMPONENT** | % |
|---|---|
| Mesalazine | 53.85% |
| Aspartame | |
| Croscarmellose sodium | |
| Citric acid | |
| Colloidal silica | |
| HPMC | |
| Magnesium stearate | |
| Eudragit L100 | |
| Methylcellulose | |
| Microcrystalline cellulose | 46.15% |
| Eudragit NE40D (contains 2% Nonoxynol 100) | |
| Polyvinylpyrrolidone | |
| Simethicone | |
| Ascorbic acid | |
| Talc | |
| Titanium dioxide | |
| Triethyl citrate | |
| Vanilla flavour (contains PEG) | |

### EXAMPLE 1:

### -CORE

The following gelled composition is prepared:

| **COMPONENT** | **AMOUNT (g)** |
|---|---|
| Polyvinylpyrrolidone | 100 |
| Polysorbate | 320 |
| Water | 580 |
| *Total* | *1000* |

dissolving Polyvinylpyrrolidone in water, to then add Polysorbate on dissolution.

Mesalazine raw material is mixed with the above-mentioned gelled composition to then extrude (500 µm net) and spheronize the compound. Immediately following spheronization, the granules obtained are dried in a Fluid Bed with inlet air at 80 °C, up to a product temperature of 50 °C. Cores having an average diameter of 480µm - 520 µm are obtained with the following dry matter composition:

| **COMPONENT** | **AMOUNT (g)** |
|---|---|
| Mesalazine | 980 |
| Polyvinylpyrrolidone | 4.76 |
| Polysorbate | 15.24 |
| *Total* | *1000* |

### -FIRST PROTECTIVE COATING

A protective membrane is prepared, comprising as follows:

| **COMPONENT** | **AMOUNT (g)** |
|---|---|
| Ethyl cellulose | 50 |
| Triethyl citrate | 1 |
| Acetone | 949 |
| *Total* | *1000* |

Dissolving Ethyl cellulose in Acetone, then adding Talc on dissolution.

The protective membrane is sprayed onto the previously obtained cores using a Glatt Fluid Bed with Wurster insert, up to a weight increase of 1% with respect to the initial weight of the pellets, thus obtaining a compound with a Mesalazine content of 97.03%.

### -SECOND GASTRO-RESISTANT COATING

A protective membrane is prepared, comprising as follows:

| **COMPONENT** | **AMOUNT (g)** |
|---|---|
| Eudragit FS 30 D | 180 |
| Polysorbate | 3 |
| Triethyl citrate | 9 |
| Glyceryl monostearate | 7 |

| | |
|---|---|
| Water | 801 |
| *Total* | *1000* |

Homogenising Polysorbate, Triethyl citrate and Glyceryl monostearate in water at 80 °C, then adding Eudragit once the compound has cooled.

The gastro resistant membrane is sprayed onto the previously obtained cores using a Glatt Fluid Bed with Wurster insert, up to a weight increase of 7% with respect to the initial weight of the pellets, thus obtaining a compound with a Mesalazine content of 90.68%.

The Finished Product has the following characteristics:

| | | |
|---|---|---|
| -Assay | 90.68 % | |
| -Average diameter | d(50) = 470 µm | d(90) =490 µm |
| -Density | 0.84 g/ml | |

The multilayer pellets thus obtained are analysed *in-vitro* in HCI 0.1N / 750 ml for 2 h, and then adjusted to pH 7.2 /1000 ml; the following dissolution profile is obtained:

| **TIMEFRAMES** | **PERCENTAGE RELEASE of the multi-layer pellets according to the invention** | **PERCENTAGE RELEASE of SALOFALK® GRANU-STIX®** | **DISSOLUTION SPECIFICATIONS** |
|---|---|---|---|
| 2h in HCl 0.1N | 0% | 0.5% | < 10% |
| *pH ADJUSTMENT* | | | |
| 30' in pH 7.2 | 68.1% | 61.4% | N.L.T. 60% |
| 60' in pH 7,2 | 98.0% | 86.9% | N.L.T. 85% |

Stability studies of the pellets according to the present invention are set up, in a climatic chamber with Temperature of 25°C and Relative Humidity of 60%, to verify the development of impurities. After 3 to 6 months the following results are obtained and are compared with the stability results of a formulation obtained according to traditional composition and production methods:
- *analysis at 3 months (25°C*-*60% RH):*

| **IMPURITIES** | **LIMITS** | **FORMULATION according to the invention** | **CLASSIC SALOFALK® GRANU-STIX® FORMULATION** |
|---|---|---|---|
| ***Known*** | 0.5% | 0.2% | 0.4% |
| ***Unknown*** | 1% | 0.3% | 1.3% |
| ***Totals*** | 1.5% | 0.5% | 1.7% |

- *analysis at 6 months (25°C* - *60% RH):*

| **IMPURITIES** | **LIMITS** | **FORMULATION according to the invention** | **CLASSIC FORMULATION** |
|---|---|---|---|
| ***Known*** | 0.5% | 0.4% | 0.6% |
| ***Unknown*** | 1% | 0.6% | 1.1% |
| ***Totals*** | 1.5% | 1.0% | 1.8% |

### EXAMPLE 2:

### -CORE

The following gelled composition is prepared:

| **COMPONENT** | **AMOUNT (g)** |
|---|---|
| Polyvinylpyrrolidone | 80 |
| Polysorbate | 300 |
| Water | 620 |
| *Total* | *1000* |

dissolving Polyvinylpyrrolidone in water, to then add Polysorbate on dissolution.

Mesalazine raw material is mixed with the above-mentioned solution to then extrude (400 µm net) and spheronize the compound. Immediately following spheronization, the granules obtained are dried in a fluid bed with inlet air at 80°C, up to a product temperature of 50 °C. Cores having an average diameter of 380 µm - 430 µm are obtained with the following dry matter composition:

| **COMPONENT** | **AMOUNT (g)** |
|---|---|
| Mesalazine | 990 |
| Polyvinylpyrrolidone | 2.10 |
| Polysorbate | 7.90 |
| *Total* | *1000* |

### -FIRST PROTECTIVE COATING

A protective membrane is prepared, comprising as follows:

| **COMPONENT** | **AMOUNT (g)** |
|---|---|
| Ethyl cellulose | 50 |
| Talc | 2.5 |
| Acetone | 947.5 |
| *Total* | *1000* |

Dissolving Ethyl cellulose in Acetone, then adding Talc on dissolution.

The protective membrane is sprayed onto the previously obtained cores using a GS Automatic Coating Pan, up to a 1 .2% increase in weight with respect to the initial weight of the pellets, thus obtaining a compound with a 97.83% Mesalazine content.

### -SECOND GASTRO-RESISTANT COATING

A protective membrane is prepared, comprising as follows:

| **COMPONENT** | **AMOUNT (g)** |
|---|---|
| Eudragit FS 30 D | 160 |
| Polysorbate | 3 |
| Triethyl citrate | 9 |
| Glyceryl monostearate | 7 |
| Water | 821 |
| *Tot.* | *1000* |

Homogenizing Polysorbate, Triethyl citrate and Glyceryl monostearate in water at 80°C, then adding Eudragit once the compound has cooled.

The gastro-resistant membrane is sprayed onto the previously obtained cores using a GS Automatic Coating Pan, up to a 7.5% increase in weight with respect to the initial weight of the pellets, thus obtaining a compound with a 91.00% Mesalazine content.

The Finished Product has the following characteristics:

| | | |
|---|---|---|
| -Assay | 91.00 % | |
| -Average diameter | d(50) = 386 µm | d(90) = 429 µm |
| -Density | 0.89 g/ml | |

The pellets are analysed *in-vitro* in HCl 0.1N /750 ml for 2 h, and then adjusted to pH 7.2/1000 ml; the following dissolution profile is obtained:

| **TIMEFRAMES** | **PERCENTAGE RELEASE of the multi-layer pellets according to the invention** | **DISSOLUTION SPECIFICATIONS** |
|---|---|---|
| 2h in HCI 0.1N | 0.3% | < 10% |
| *pH ADJUSTMENT* | | |
| 30' in pH 7.2 | 79.4% | N.L.T. 60% |
| 60' in pH 7,2 | 99.2% | N.L.T. 85% |

Stability studies of the pellets according to the present invention are set up, in a climatic chamber with Temperature of 25°C and Relative Humidity of 60%, to verify the development of impurities. After 3 to 6 months the following results are obtained and are compared with the stability results of a formulation obtained according to traditional composition and production methods:
- *analysis at 3 months (25 °C*-*60% RH):*

| **IMPURITIES** | **LIMITS** | **FORMULATION according to the invention** | **CLASSIC FORMULATION** |
|---|---|---|---|
| ***Known*** | 0.5% | 0.3% | 0.4% |
| ***Unknown*** | 1% | 0.2% | 1.3% |

| | | | |
|---|---|---|---|
| ***Totals*** | 1 .5% | 0.5% | 1.7% |

- *analysis at 6 months (25 °C* - *60% RH):*

| **IMPURITIES** | **LIMITS** | **FORMULATION according to the invention** | **CLASSIC FORMULATION** |
|---|---|---|---|
| ***Known*** | 0.5% | 0.4% | 0.6% |
| ***Unknown*** | 1% | 0.3% | 1.1% |
| ***Totals*** | 1.5% | 0.7% | 1.8% |

### EXAMPLE 3:

### -CORE

The following gelled composition is prepared:

| **COMPONENT** | **AMOUNT (g)** |
|---|---|
| Polyvinylpyrrolidone | 90 |
| Polysorbate | 400 |
| Water | 510 |
| *Total* | *1000* |

dissolving Polyvinylpyrrolidone in water, to then add Polysorbate on dissolution.

Mesalazine raw material is mixed with the above-mentioned solution to then extrude (600 µm net) and spheronize the compound. Immediately following spheronization, the granules obtained are dried in a Fluid Bed with inlet air at 80°C, up to a product temperature of 50°C. Cores having an average diameter of 570 µm - 640 µm are obtained with the following dry matter composition:

| **COMPONENT** | **AMOUNT (g)** |
|---|---|
| Mesalazine | 985 |
| Polyvinylpyrrolidone | 2.75 |
| Polysorbate | 12.25 |
| *Total* | *1000* |

### -FIRST PROTECTIVE COATING

A protective membrane is prepared, comprising as follows:

| **COMPONENT** | **AMOUNT (g)** |
|---|---|
| Ethyl cellulose | 50 |
| Acetone | 950 |
| *Total* | *1000* |

By dissolving Cellulose in Acetone.

The protective membrane is sprayed onto the previously obtained cores using a Glatt Fluid Bed with Wurster insert, up to a weight increase of 0.8% with respect to the initial weight of the pellets, thus obtaining a compound with a Mesalazine content of 97.72%.

### -SECOND GASTRO-RESISTANT COATING

A protective membrane is prepared, comprising as follows:

| **COMPONENT** | **AMOUNT (g)** |
|---|---|
| Eudragit FS 30 D | 180 |
| Talc | 18 |
| Triethyl citrate | 36 |
| Water | 766 |
| *Total* | *1000* |

Homogenising Triethyl citrate and Talc in water, then adding Eudragit to the compound.

The gastro resistant membrane is sprayed onto the previously obtained cores using a Glatt Fluid Bed with Wurster insert, up to a weight increase of 7% with respect to the initial weight of the pellets, thus obtaining a compound with a Mesalazine content of 91.33%.

The Finished Product has the following characteristics:

| | | |
|---|---|---|
| - Assay | 91.33 % | |
| -Average diameter | d(50) = 586 µm | d(90) = 624 µm |
| -Density | 0.81 g/ml | |

The pellets are analysed *in-vitro* in HCl 0.1N / 750 ml for 2 h, and then adjusted to pH 7.2/1000 ml; the following dissolution profile is obtained:

| **TIMEFRAMES** | **PERCENTAGE RELEASE of the multi-layer pellets according to the invention** | **DISSOLUTION SPECIFICATIONS** |
|---|---|---|
| 2h in HCl 0.1 N | 1,4% | < 10% |
| *pH ADJUSTMENT* | | |
| 30' in pH 7.2 | 6 6.4% | N.L.T. 60% |
| 60' in pH 7,2 | 95.2% | N.L.T. 85% |

Stability studies of the pellets according to the present invention are set up, in a climatic chamber with Temperature of 25°C and Relative Humidity of 60%, to verify the development of impurities. After 3 to 6 months the following results are obtained and are compared with the stability results of a formulation obtained according to traditional composition and production methods:
- *analysis at 3 months (25 °C* - *60% RH):*

| **IMPURITIES** | **LIMITS** | **FORMULATION according to the invention** | **CLASSIC FORMULATION** |
|---|---|---|---|
| ***Known*** | 0.5% | 0.2% | 0.4% |
| ***Unknown*** | 1% | 0.6% | 1.3% |
| ***Totals*** | 1.5% | 0.8% | 1.7% |

- *analysis at* 6 *months (25 °C* - *60% RH):*

| **IMPURITIES** | **LIMITS** | **FORMULATION according to the invention** | **CLASSIC FORMULATION** |
|---|---|---|---|
| ***Known*** | 0.5% | 0.3% | 0.6% |
| ***Unknown*** | 1% | 0.6% | 1.1% |
| ***Totals*** | 1.5% | 0.9% | 1.8% |

## Claims

1. Granules containing Mesalazine as Active Pharmaceutical Ingredient (API), said granules consisting of API in mixture with a dried gelled composition in a ratio from 97:3 to 99:1, referred to the dry portion of the composition; said granules being obtained by extrusion, spheronization and drying of a mixture of the API with a gelled composition consisting of a mixture of 5-10% Polyvinyl pyrrolidone, 20-40% Polysorbate and 45-75% Water, where % refers to the percentages by weight with respect to the total weight of the gelled composition.

2. Granules according to the preceding claim having average size from 0.4 mm to 2 mm.

3. A pharmaceutical formulation comprising the granules according to any one of the preceding claims.

4. Multi-layer pellets comprising the granules according to any one of claims 1-2 as the inner core.

5. Multi-layer pellets according to the preceding claim, said pellets comprising:
an inner core consisting of the granules according to any one of claims 1-2;
a first inner coating membrane, surrounding and contacting the core, said first membrane being pH-independent and comprising a polymer derived from cellulose, dissolved in non-aqueous solvent;
a second outer coating membrane, surrounding the first membrane, said second membrane being gastroprotective pH-dependent and comprising a polymer derivative of methacrylic acid selected from the anionic polymers with methacrylic acid as the functional group.

6. Multi-layer pellets according to the preceding claim, wherein the first membrane comprises Ethyl cellulose having 3-110 mPa.s (cPs) viscosity, and the non-aqueous solvent is selected from Acetone, Ethanol and mixtures thereof; wherein the ethyl cellulose viscosity was measured on 5% solutions in Toluene/Ethanol (80%:20%) measured at 25°C in a Ubbelohde viscometer.

7. Multi-layer pellets according to any one of claims 5-6, wherein the second membrane comprises a polymer selected from Eudragit L100-55, Eudragit L 30 D-55, Eudragit L100, Eudragit L12,5, Eudragit S100, Eudragit S12,5 , Eudragit FS 30 D or mixtures thereof.

8. A pharmaceutical formulation for oral administration of Mesalazine, said formulation comprising the pellets according to any one of claims 5-7.

9. A pharmaceutical formulation according to the preceding claim, said formulation being in the form of sachet, tablet or capsule.

10. A process for preparing the granules according to any one of claims 1-2, said process comprising preparing the gelled composition by dissolving Polyvinyl pyrrolidone in water first; and then, upon achieved dissolution, adding Polysorbate to achieve the gelification; the above-mentioned gelled composition is then added to and mixed with Mesalazine, and finally the compound is extruded, spheronized and dried.

11. A process for preparing the multi-layer pellets according to any one of claims 4-7, said process comprising:
coating the inner core with the first membrane in Fluid Bed or in Coating Pan until achieving an increase in weight from 0.2% to 2% of dry portion with respect to the weight of the cores;
coating with the second membrane in Fluid Bed or in Coating Pan until achieving an increase in weight from 5% to 15% of dry portion with respect to the weight of the cores.

## Patentansprüche

1. Granulat, das Mesalazin als aktiven pharmazeutischen Bestandteil (API) enthält, wobei das Granulat aus API in Mischung mit einer getrockneten gelierten Zusammensetzung in einem Verhältnis von 97:3 bis 99:1 besteht, bezogen auf den trockenen Anteil der Zusammensetzung; wobei das Granulat durch Extrusion, Spheronisierung und Trocknung eines Gemisches des API mit einer gelierten Zusammensetzung erhalten wird, die aus einer Mischung von 5-10% Polyvinylpyrrolidon, 20-40% Polysorbat und 45-75% Wasser besteht, wobei % sich auf die Gewichtsprozentsätze bezogen auf das Gesamtgewicht der gelierten Zusammensetzung bezieht.

2. Granulat nach dem vorhergehenden Anspruch mit einer durchschnittlichen Größe von 0,4 mm bis 2 mm.

3. Pharmazeutische Formulierung, umfassend das Granulat nach einem der vorhergehenden Ansprüche.

4. Mehrschichtige Pellets, umfassend das Granulat nach einem der Ansprüche 1 bis 2 als den inneren Kern.

5. Mehrschichtige Pellets nach dem vorhergehenden Anspruch, wobei die Pellets umfassen:
einen inneren Kern, der aus dem Granulat nach einem der Ansprüche 1 bis 2 besteht;
eine erste innere Beschichtungsmembran, die den Kern umgibt und berührt, wobei die erste Membran pH-unabhängig ist und ein Polymer umfasst, das von Cellulose abgeleitet ist, gelöst in nicht-wässrigem Lösungsmittel;
eine zweite äußere Umhüllungsmembran, die die erste Membran umgibt, wobei die zweite Membran gastroprotektiv pH-abhängig ist und ein polymeres Derivat von Methacrylsäure, ausgewählt aus den anionischen Polymeren mit Methacrylsäure als funktionelle Gruppe, umfasst.

6. Mehrschichtige Pellets nach dem vorhergehenden Anspruch, wobei die erste Membran Ethylcellulose mit einer Viskosität von 3-110 mPa.s (cPs) umfasst und das nicht-wässrige Lösungsmittel ausgewählt ist aus Aceton, Ethanol und Gemischen davon; wobei die Ethylcellulose-Viskosität an 5%-igen Lösungen in Toluol/Ethanol (80%:20%) gemessen bei 25ºC in einem Ubbelohde-Viskosimeter gemessen wurde.

7. Mehrschichtige Pellets nach einem der Ansprüche 5 bis 6, wobei die zweite Membran ein Polymer umfasst, ausgewählt aus Eudragit L100-55, Eudragit L 30 D-55, Eudragit L100, Eudragit L12,5, Eudragit S100, Eudragit S12,5, Eudragit FS 30 D oder Mischungen davon.

8. Pharmazeutische Formulierung zur oralen Verabreichung von Mesalazin, wobei die Formulierung die Pellets nach einem der Ansprüche 5 bis 7 umfasst.

9. Pharmazeutische Formulierung nach dem vorhergehenden Anspruch, wobei die Formulierung in Form eines Beutels, einer Tablette oder einer Kapsel vorliegt.

10. Verfahren zur Herstellung des Granulats nach einem der Ansprüche 1 bis 2, wobei das Verfahren umfasst: zuerst Herstellen der gelierten Zusammensetzung durch Auflösen von Polyvinylpyrrolidon in Wasser; und dann, nach der erreichten Auflösung, Zugabe von Polysorbat, um die Gelierung zu erreichen; wobei die oben erwähnte gelierte Zusammensetzung dann zu Mesalazin gegeben und damit gemischt wird und schließlich die Verbindung extrudiert, spheronisiert und getrocknet wird.

11. Verfahren zur Herstellung der mehrschichtigen Pellets nach einem der Ansprüche 4 bis 7, wobei das Verfahren umfasst:
Beschichten des inneren Kerns mit der ersten Membran im Wirbelbett oder in der Dragierpfanne, bis eine Gewichtszunahme von 0,2% bis 2% des trockenen Anteils in Bezug auf das Gewicht der Kerne erreicht ist;
Beschichten mit der zweiten Membran im Wirbelbett oder in der Dragierpfanne, bis eine Gewichtszunahme von 5% bis 15% des trockenen Anteils in Bezug auf das Gewicht der Kerne erreicht ist.

## Revendications

1. Granulés contenant de la mésalazine en tant que principe pharmaceutique actif (API pour Active Pharmaceutical Ingredient), lesdits granulés se composant d'API en mélange avec une composition gélifiée séchée selon un rapport de 97:3 à 99:1, dénommée partie sèche de la composition ; lesdits granulés étant obtenus par extrusion, sphéronisation et séchage d'un mélange de l'API avec une composition gélifiée se composant d'un mélange de 5 à 10 % de polivinylpyrrolidone, de 20 à 40 % de polysorbate et de 45 à 75 % d'eau, le % faisant référence aux pourcentages en poids par rapport au poids total de la composition gélifiée.

2. Granulés selon la revendication précédente, ayant une taille moyenne de 0,4 mm à 2 mm.

3. Formulation pharmaceutique comprenant les granulés selon l'une quelconque des revendications précédentes.

4. Pastilles multicouches comprenant les granulés selon l'une quelconque des revendications 1 à 2 en tant que noyau interne.

5. Pastilles multicouches selon la revendication précédente, lesdites pastilles comprenant :
un noyau interne se composant des granulés selon l'une quelconque des revendications 1 à 2 ;
une première membrane de revêtement interne, entourant et en contact avec le noyau, ladite première membrane étant indépendante du pH et comprenant un polymère dérivé de la cellulose, dissous dans un solvant non aqueux ;
une seconde membrane de revêtement externe, entourant la première membrane, ladite seconde membrane étant gastroprotectrice, dépendante du pH et comprenant un polymère dérivé de l'acide méthacrylique choisi parmi les polymères anioniques avec de l'acide méthacrylique en tant que groupe fonctionnel.

6. Pastilles multicouches selon la revendication précédente, dans lesquelles la première membrane comprend de l'éthylcellulose ayant une viscosité de 3 à 110 mPa.s (cPs) et le solvant non aqueux est choisi parmi l'acétone, l'éthanol et des mélanges de ceux-ci ; dans lesquelles la viscosité de l'éthylcellulose est mesurée sur des solutions à 5 % dans un mélange de toluène/éthanol (80 %:20 %) mesurées à 25 °C dans un viscosimètre Ubbelohde.

7. Pastilles multicouches selon l'une quelconque des revendications 5 à 6, dans lesquelles la seconde membrane comprend un polymère choisi parmi l'Eudragit L100-55, l'Eudragit L 30 D-55, l'Eudragit L100, l'Eudragit L12,5, l'Eudragit S100, l'Eudragit S12,5, l'Eudragit FS 30 D ou des mélanges de ceux-ci.

8. Formulation pharmaceutique destinée à l'administration orale de mésalazine, ladite formulation comprenant les pastilles selon l'une quelconque des revendications 5 à 7.

9. Formulation pharmaceutique selon la revendication précédente, ladite formulation étant sous la forme d'un sachet, d'un comprimé ou d'une gélule.

10. Procédé de préparation des granulés selon l'une quelconque des revendications 1 à 2, ledit procédé comprenant la préparation de la composition gélifiée par dissolution de polyvinylpyrrolidone dans de l'eau en premier ; et ensuite, lorsque la dissolution a été atteinte, par ajout de polysorbate pour obtenir la gélification ; la composition gélifiée mentionnée ci-dessus est ensuite ajoutée à et mélangée avec la mésalazine, et enfin le composé est extrudé, sphéronisé et séché.

11. Procédé de préparation des pastilles multicouches selon l'une quelconque des revendications 4 à 7, ledit procédé comprenant :
le revêtement du noyau interne avec la première membrane dans un lit fluid ou dans une cuve d'enrobage jusqu'à l'obtention d'une augmentation de poids de 0,2 % à 2 % de la partie sèche par rapport au poids des noyaux ;
le revêtement avec la seconde membrane dans un lit fluid ou dans une cuve d'enrobage jusqu'à l'obtention d'une augmentation de poids de 5 % à 15 % de la partie sèche par rapport au poids des noyaux.
